# EUROPÄISCHE PATENTSCHRIFT

(11) **EP 2 029 041 B1**
(45) Veröffentlichungstag und Bekanntmachung des Hinweises auf die Patenterteilung: **27.10.2010**
(21) Anmeldenummer: 07764608.1
(22) Anmeldetag: 11.06.2007
(51) Int. Cl.: A61B 18/22

(54) **HANDSTÜCK FÜR INSBESONDERE MEDIZINISCHE LASERANWENDUNGEN**
HANDPIECE INTENDED PARTICULARLY FOR MEDICAL LASER APPLICATIONS
PIÈCE À MAIN, EN PARTICULIER POUR APPLICATIONS MÉDICALES UTILISANT UN LASER

(30) Priorität: 13.06.2006 DE 102006027624
(43) Veröffentlichungstag der Anmeldung: 04.03.2009
(73) Patentinhaber: elexxion AG, 78315 Radolfzell (DE)
(72) Erfinder: SCHÄFER, Olaf, 78224 Singen (DE)
(74) Vertreter: Weiss, Peter
(86) Internationale Anmeldenummer: PCT/EP2007/005125
(87) Internationale Veröffentlichungsnummer: WO 2007/144124

(56) Entgegenhaltungen:
- WO-A-93/04727
- WO-A-2006/029877
- DE-A1-102004 055 412
- DE-B3- 10 239 950
- US-A- 6 011 889

## Beschreibung

Die Erfindung betrifft ein Handstück nach dem Oberbegriff des Anspruchs 1, wie aus der DE 10239950 B3 bekannt.

### Stand der Technik

In vielen medizinischen Bereichen werden heute Laser für Operationen bzw. Behandlungen verwendet, wobei nur beispielsweise auf Augenoperation, Prostatabehandlungen und die Verwendung im dentalen Bereich hingewiesen wird. Vor allem dient der Laser zu Schneid- bzw. Schabzwecken, aber auch vor allem zum Koagulieren von Gewebe. Im ersteren Fall wird in der Regel ein Laserstrahl über eine gewisse Entfernung ausgesandt, so dass der eigentlich Laser oder das entsprechende Handstück nicht direkt mit dem Operationsumfeld in Berührung kommt. Ein derartiges Handstück wird beispielsweise in der DE 699 20 236 T2 gezeigt. Entscheidende Hindernisse für diese Laserstrahlung sind jedoch die Eindringtiefe und Streuung im biologischen Gewebe.

Die DE 10 2004 055 412 A1 beschreibt ein Lasersystem in Verbindung mit einem medizinischen Gefässaplikator.

Gerade zur Behandlung von biologischem Gewebe kann jedoch ein Laser sehr gut eingesetzt werden, bei dem die Laserfaser mit dem Gewebe in Berührung gebracht wird. Die Schneidwirkung wird dabei durch das distale Ende der Laserfaser bewirkt, das, von der Laserstrahlung aufgeheizt, das berührte Gewebearial verdampft. Durch die entsprechende Koagulation findet auch eine Blutstillung statt. Nachteilig hat sich hier herausgestellt, dass beim direkten Gewebekontakt die Spitze der Laserfaser verschmutzt und auch verschliessen wird. Die Verschmutzung durch verkohlte Blut- und Gewebepartikel mindert bzw. verhindert ein Austreten der Strahlung an der Faserspitze. Aus diesem Grunde muss die Laserfaser, zumal sie auch durch ihre weit herausstehende Länge einer mechanischen Beschädigung ausgesetzt ist, sehr häufig ersetzt werden. Dies ist teuer und zeitaufwendig.

Um diesem Nachteil zu begegnen, schlägt die DE 42 09 926 A1 vor, dass eine wechselseitige oder gleichzeitige Anwendung von Laserstrahlung zum Schneidung und Koagulieren mit zweier konzentrisch angeordneter Lichtwellenleiter erreicht wird, in die Laserstrahlung mit variablem Intensitätsverteilungsprofil so eingekoppelt wird, dass der grössere Teil der Strahlleistung entweder auf die Innen- oder die Aussenfaser oder auf beide in jeweils gewünschtem Masse trifft. D. h., die Laserfaser wird in eine Innenfaser und eine Aussenfaser aufgeteilt, wobei diesen beiden Fasern eine im Handstück vorhandene Laserfaser mit Linsenoptik zugeordnet ist. Diese im Inneren angeordnete Laserfaser wird von der Kombination aus Innen- und Aussenfaser so entfernt, dass entweder die Innenfaser oder die Aussenfaser mit der Laserstrahlung versorgt wird.

Abgesehen davon, dass diese Vorrichtung kompliziert zu bedienen ist, bleibt der aus der Kanalausmündung austretende Bereich der Laserfaser einem mechanischen Verschleiss und der oben angesprochenen Verschmutzung ausgesetzt.

### Aufgabe

Aufgabe der vorliegenden Erfindung ist es, auf einfache Art einen Verschleiss der aus der Kanalausmündung austretenden Laserfaserspitze zu verringern.

### Lösung der Aufgabe

Zur Lösung der Aufgabe führt der kennzeichnende Teil des Anspruchs 1.

Das bedeutet, dass bei einer medizinischen Anwendung die Laserfaser nur mit dem geringstmöglichen Bereich aus der Kanalausmündung herausschaut. Damit ist sie einem geringeren mechanischen Verschleiss ausgesetzt, da es schwieriger ist, einen kurzen Faserabschnitt zu brechen, als einen langen Faserabschnitt. Ferner wird auch nur dieser kurze Faserabschnitt verschmutzt. Ist er so verschmutzt, dass kein Strahl mehr hindurchtritt, kann er beispielsweise ausserhalb des Operationsfeldes mechanisch abgebrochen werden. Durch diese beiden Vorteile kann die Anwendungsdauer der heute eingesetzten Laserfaser wesentlich erhöht und damit auch die Kosten verringert werden.

In einem Ausführungsbeispiel bestünde die Möglichkeit, bekannte bisher einstückige Handstücke nach wie vor zu verwenden und das Einsetzen einer Laserfaser in dieses Handstück zu begrenzen. Die Laserfaser müsste dann so ausgestaltet werden, dass sie nach und nach durch das Handstück nachgeschoben werden kann. Dies erscheint kompliziert, ist aber denkbar.

In einem bevorzugten Ausführungsbeispiel soll dagegen das Handstück in seiner Dimensionierung veränderbar ausgestaltet werden. Das bedeutet, dass die Laserfaser mit ihrer Linsenoptik an Ort und Stelle in dem Handstück verbleibt und so die Optik in keinem Fall gestört wird. Dies gewährleistet die Funktionssicherheit des Lasers. Die Verbindung von Laserfaser zu Optik bleibt unverändert erhalten.

Die Veränderung der Dimensionierung des Lasers kann einmal dadurch geschehen, dass das Handstück aus zumindest zwei Teilen hergestellt wird, deren Lage relativ zueinander veränderbar ist. Zu dieser Lageveränderung werden zwei Möglichkeiten bevorzugt. Die eine Möglichkeit besteht darin, dass zwei Teile axial zueinander verschoben werden. D. h., das Handstück wird quasi gestreckt, wodurch ein Teil der aus der Kanalausmündung austretenden Laserfaser in den Innenkanal eingezogen wird.

Zu diesen Zweck gehen die beiden Teile bevorzugt miteinander eine Rastverbindung ein, wobei diese Rastverbindung beliebig ausgestaltet sein kann. In einem bevorzugten Ausführungsbeispiel wird das Handstück in ein Anschlussstück und ein Frontstück unterteilt. Diese gehen miteinander eine Steckverbindung ein, d. h., das Anschlussstück greift mit einer Steckhülse in eine Aufschubhülse des Frontstückes ein. Selbstverständlich kann diese Anordnung auch umgekehrt sein. Um nun die Rastverbindung zu vereinfachen, sind auf dem Umfang der Steckhülse eine Mehrzahl von zinnenartigen Rastringen ausgeformt, die mit elastischen Elementen, insbesondere Gummiringe zusammenwirken, die von entsprechenden Ringnuten in der Aufschubhülse aufgenommen sind. Werden nun Anschlussstück und Frontstück auseinandergezogen, so quetschen sich die Gummiringe über die zinnenartigen Rastringe und entspannen sich dann in die Zwischenräume zwischen den Rastringen, sodass dort eine Festlegung des Frontstückes gegenüber dem Anschlussstück erfolgt.

Als weitere Möglichkeit einer Rastzahnung ist daran gedacht, bspw. dem Frontstück oder auch dem Anschlussstück eine Rastzahnung zuzuordnen, die mit einem von aussen her zu betätigenden Rasthebel zusammenwirkt. Dieser Rasthebel ist bevorzugt als Kipphebel ausgestaltet, wobei er durch eine entsprechende Druckfeder in Rastlage gehalten wird.

Denkbar wäre auch, in diesen Bereich eine Gewindeverzahnung vorzusehen, sodass beispielsweise das Anschlussstück aus dem Frontstück herausgeschraubt werden könnte. Dadurch besteht jedoch die Gefahr des Verdrillens der Laserfaser, sodass dies unerwünscht ist. In diesem Fall wäre es besser, die Laserfaser aus dem Innenkanal herauszuziehen, das Gewinde zu drehen und danach die Laserfaser wieder einzusetzen. Dies ist aber umständlich und erfordert Zeit.

Ein derartiges Verdrehen von zwei Teilen des Handstücks gegeneinander ist jedoch nahe der Ausmündung der Laserfaser möglich, da dort ein Verdrehen eines Schraubeinsatzes die Laserfaser wenig beeinflusst. Damit wäre dort die Anordnung eines zweiten Ausführungsbeispiels möglich, wobei der Schraubeinsatz mit einem Aussengewinde ein Innengewinde in dem Frontstück kämmt. Dieses Ausführungsbeispiel soll vor allem dann gewählt werden, wenn die Veränderung der Länge des aus der Kanalausmündung austretenden Faserstücks sehr genau bestimmt werden muss, da dies besonders gut durch den Schraubeinsatz möglich ist.

Um den Schraubeinsatz in seiner gewünschten Position festzulegen, ist ein Klemmelement, beispielsweise ein Madenschrauber od. dgl. vorgesehen.

### Figurenbeschreibung

Weitere Vorteile, Merkmale und Einzelheiten der Erfindung ergeben sich aus den nachfolgenden Beschreibung bevorzugter Ausführungsbeispiele sowie anhand der Zeichnung; diese zeigt in
Figur 1 einen Längsschnitt durch ein erfindungsgemässes Handstück für medizinische Laseranwendungen;
Figur 2 einen Längsschnitt durch das Handstück gemäss Figur 1 in einer weiteren Gebrauchslage;
Figur 3 einen teilweise dargestellten Längsschnitt durch einen Bereich eines Frontstücks eines Handstücks für medizinische Laseranwendung in einer weiteren Ausführungsform;
Figur 4 einen Ausschnitt aus einem Längsschnitt entsprechend Figur 3 in einer weiteren Gebrauchslage;
Figur 5 einen Ausschnitt aus einem Längsschnitt eines weiteren Ausführungsbeispiels eines Handstücks für medizinische Laseranwendung.

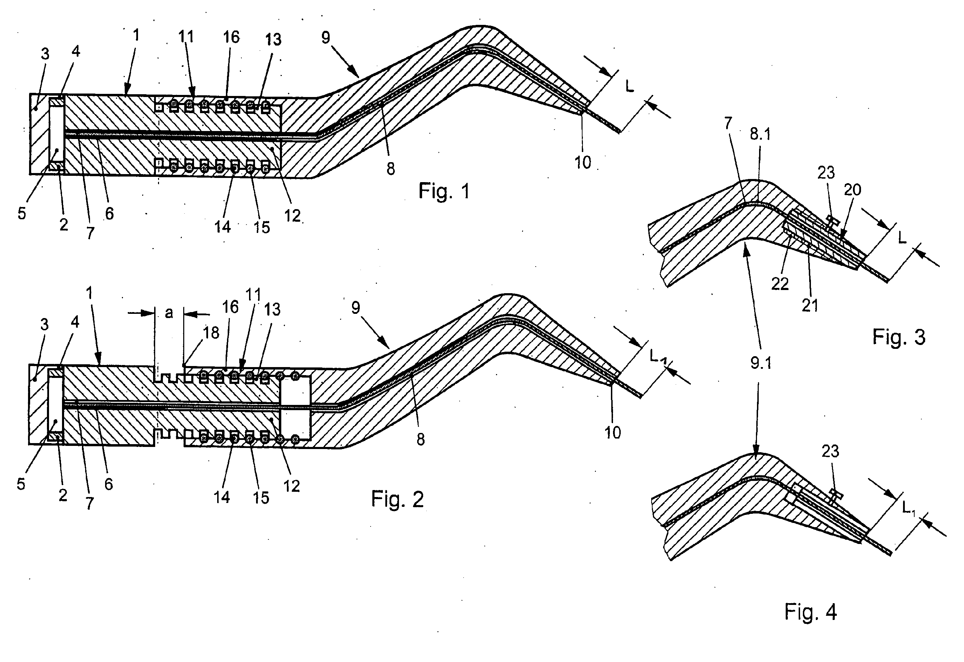

Gemäss Figur 1 weist ein erfindungsgemässes Handstück für medizinische Laseranwendungen ein zylindrisches Anschlussstück 1 auf, welches linksseitig einen Ringkragen 2 ausbildet, der mit einem nicht näher gekennzeichneten Aussengewinde versehen ist. Dieses Aussengewinde wirkt mit einem ebenfalls nicht näher gekennzeichneten Innengewinde eine Verschlusskappe 3 zusammen, die auf den Ringkragen 2 aufgeschraubt ist. Die Verschlusskappe 3 bildet zusammen mit den Anschlussstück 1 einen Ringraum 4 zur Aufnahme eines Faseranschlusses 5 bzw. Linsenoptik aus, an die eine in einem Axialkanal 6 geführte Laserfaser 7 anschliesst.

Der Axialkanal 6 geht in einen Kanal 8 über, der ein Frontstück 9 des Handstücks durchzieht. Der Kanal 8 ist in Bögen durch das Frontstück 9 geführt, je nachdem, wie das Frontstück 9 gekrümmt ist. Er bildet zusammen mit dem Axialkanal 6 einen Innenkanal zur Führung der Laserfaser 7 aus, die aus einer Kanalausmündung 10 an der Spitze des Frontstücks 9 austritt und über die Kanalausmündung 10 um eine Länge L hervorsteht.

Das Frontstück 9 geht mit dem Anschlussstück 1 eine Rastverbindung 11 ein. Zu diesem Zweck sind einer Steckhülse 12 des Anschlussstücks 1 eine Mehrzahl von zinnenartigen Rastringen 13 auf deren Umfang angeformt, die mit Gummiringen 14 in entsprechenden Ringnuten 15 in der Innenfläche einer Aufschubhülse 16 zusammenwirken, welche von dem Frontstück 9 ausgebildet wird.

Die Funktionsweise der vorliegenden Erfindung ist folgende und wird im Zusammenhang mit Figur 2 erläutert:
In Figur 1 ist die Aufschubhülse 16 vollständig über die Steckhülse 2 geschoben, sodass die Laserfaser 7 in ihrer maximalen Länge L aus der Kanalausmündung 10 hervorsteht. Soll nun diese Länge L verringert werden, beispielsweise zu Beginn einer Laserbehandlung, damit nur ein geringer Bereich der Spitze der Laserfaser 7 verschmutzt oder verschliessen wird, so werden Anschlussstück 1 und Frontstück 9 durch Ziehen auseinandergezogen, wobei die Gummiringe 14 durch die Rastringe 13 gequetscht werden und dann wieder in den Zwischenräumen zwischen den Rastringen 13 sich entspannen können. Werden z. B. zwei zinnenartige Rastringe 13 durch die Gummiringe 14 übersprungen, wie dies in Figur 2 dargestellt ist, so ergibt dies einen Abstand a einer Innenkante 17 des Anschlussstücks 1 von einer Stirnkante 18 der Aufschubhülse 16. Gleichzeitig verringert sich die Länge des aus der Kanalausmündung 10 hervorstehenden Endes der Laserfaser 7 auf eine Länge L₁.

Wird im Laufe der Zeit eine grössere Länge der Laserfaser benötigt, wird das Frontstück 9 rastringweise weiter über die Steckhülse 12 geschoben, sodass immer die gewünschte Länge aus der Kanalausmündung hervorsteht.

Bei einem weiteren Ausführungsbeispiel der Erfindung gemäss den Figuren 3 und 4 ist daran gedacht, in die Spitze des Frontstückes 9 einen Schraubeinsatz 20 einzusetzen. Dieser Schraubeinsatz 20 weist ein Aussengewinde 21 auf, welches mit einem Innengewinde 22 in dem Frontstück 9.1 zusammenwirkt. Der Schraubeinsatz 20 bildet auch einen Kanalbereich 8.1 für das Führen der Laserfaser 7 aus.

Soll die Länge L zu der Länge L₁, wie in Figur 4 gezeigt, verkleinert werden, so wird eine Klemmschraube 23 gelöst und der Schraubeinsatz 20 kann ein Stück weit aus dem Frontstück 9.1 herausgeschraubt werden.

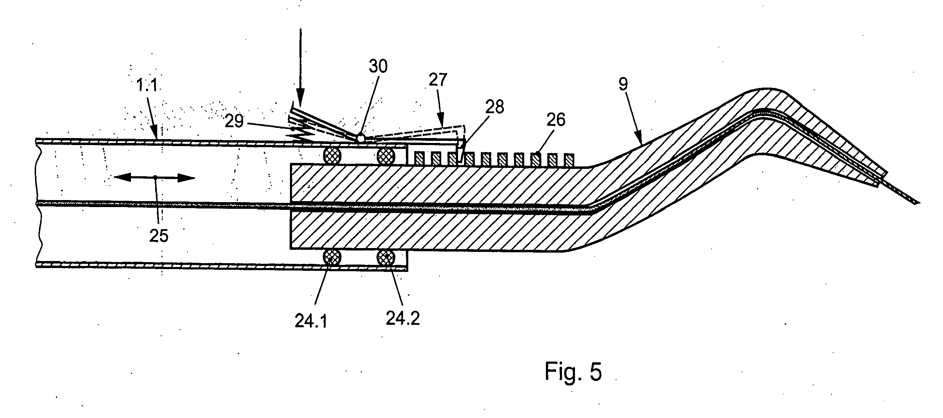

In einem weiteren Ausführungsbeispiel der Erfindung gemäss Figur 5 ist das Frontstück 9 in ein hülsenförmiges Anschlussstück 1.1 eingeschoben. Zum Zwecke der Abdichtung und Lagerung sind zwei Gleitringe 24.1 und 24.2 zwischen Frontstück 9 und Anschlussstück 1.1 vorgesehen. Das Frontstück 9 ist in dem hülsenförmigen Anschlussstück 1.1 entsprechend dem Doppelpfeil 25 verschiebbar.

Erfindungsgemäss sitzt auf dem Frontstück 1 eine Rastzahnung 26 auf, die mit einem Rasthebel 27 zusammenwirkt. Dieser Rasthebel 27 ist als Kipphebel ausgebildet und besitzt an seinem einen freien, der Rastzahnung 26 zugewandten Ende einen Rastzahn 28, während gegen sein anderes freies Ende eine Druckfeder 29 sich gegen dieses freie Ende und das Anschlussstück 1.1 abstützt. Zwischen den beiden freien Enden besitzt der Rasthebel 27 eine gelenkige Verbindung 30 mit dem Anschlussstück 1.1.

Wird mit dem Finger auf das eine Ende über der Druckfeder 29 gedrückt, so kippt der Rasthebel 27 um die gelenkige Verbindung 30 und der Rastzahn 28 löst sich aus der Rastzahnung 26. Damit kann das Frontstück 9 in Richtung des Doppelpfeils 25 innerhalb des hülsenförmigen Anschlussstückes 1.1 verschoben werden.

Im Rahmen der Erfindung liegt selbstverständlich auch die Möglichkeit, dass die gezeigten Einrichtungen zum Verändern der Länge L miteinander an einem Handstück vorgesehen sind.

### Bezugszeichenliste

| | | | | | |
|---|---|---|---|---|---|
| 1 | Anschlussstück | 34 | | 67 | |
| 2 | Ringkragen | 35 | | 68 | |
| 3 | Verschlusskappe | 36 | | 69 | |
| 4 | Ringraum | 37 | | 70 | |
| 5 | Faseranschluss | 38 | | 71 | |
| 6 | Axialkanal | 39 | | 72 | |
| 7 | Laserfaser | 40 | | 73 | |
| 8 | Kanal | 41 | | 74 | |
| 9 | Frontstück | 42 | | 75 | |
| 10 | Kanalausmündung | 43 | | 76 | |
| 11 | Rastverbindung | 44 | | 77 | |
| 12 | Steckhülse | 45 | | 78 | |
| 13 | Rastring | 46 | | 79 | |
| 14 | Gummiring | 47 | | | |
| 15 | Ringnut | 48 | | | |
| 16 | Aufschubhülse | 49 | | | |
| 17 | Innenkante | 50 | | | |
| 18 | Stirnkante | 51 | | | |
| 19 | | 52 | | a | Abstand |
| 20 | Schraubeinsatz | 53 | | L | Länge |
| 21 | Aussengewinde | 54 | | L₁ | Länge |
| 22 | Innengewinde | 55 | | | |
| 23 | Klemmschrauben | 56 | | | |
| 24 | Gleitring | 57 | | | |
| 25 | Doppelpfeil | 58 | | | |
| 26 | Rastzahnung | 59 | | | |
| 27 | Rasthebel | 60 | | | |
| 28 | Rastzahn | 61 | | | |
| 29 | Druckfeder | 62 | | | |
| 30 | gelenkige Verbindung | 63 | | | |
| 31 | | 64 | | | |
| 32 | | 65 | | | |
| 33 | | 66 | | | |

## Patentansprüche

1. Handstück für insbesondere medizinische Laseranwendungen mit einem Innenkanal (6, 8, 8.1), in den eine Laserfaser (7) eingesetzt ist, die mit einem freien Endabschnitt einer Länge (L) über eine Kanalausmündung (10) hervorsteht, wobei die Länge (L) der aus der Kanalausmündung (10) austretenden Laserfaser (7) veränderbar ist,
wobei
es zumindest aus zwei Teilen (1, 1.1, 9, 9.1, 20) besteht, deren Lage relativ zueinander veränderbar ist, **dadurch gekennzeichnet, dass** auf oder in einem Teil eine Rastzahnung (26) vorgesehen ist, in welche ein betätigbarer Rasthebel (27), welcher als Kipphebel ausgebildet ist, der an dem anderen Teil festliegt, eingreift.

2. Handstück nach Anspruch 1, **dadurch gekennzeichnet, dass** die Lageänderung durch ein axiales Verschieben von zumindest zwei Teilen (1, 1.1, 9) im Verhältnis zueinander erfolgt.

3. Handstück nach einem der vorigen Ansprüche, **dadurch gekennzeichnet, dass** ein zylindrisches Anschlussstück (1) auf dem Umfang einer Steckhülse (12) eine Mehrzahl von zinnenartigen Rastringen (13) aufweist, denen in einer Aufschubhülse (16) eines Frontstückes (9) elastische Elemente (14) zugeordnet sind, welche zwischen die Rastringe (13) greifen.

4. Handstück nach Anspruch 3, **dadurch gekennzeichnet, dass** die elastischen Elemente Gummiringe (14) sind, die in Ringnuten (15) in der Aufschubhülse (16) liegen.

5. Handstück nach einem der vorigen Ansprüche, **dadurch gekennzeichnet, dass** die Lageänderung durch ein Verdrehen von zumindest zwei Teilen (9.1, 20) zueinander erfolgt.

6. Handstück nach einem der vorigen Ansprüche, **dadurch gekennzeichnet, dass** in ein Frontstück (9.1) ein Schraubeinsatz (20) eingesetzt ist, der auch die Kanalausmündung (10) aufweist.

7. Handstück nach Anspruch 6, **dadurch gekennzeichnet, dass** der Schraubeinsatz (20) mit einem Aussengewinde (21) ein Innengewinde (22) in dem Frontstück (9.1) kämmt.

8. Handstück nach Anspruch 6 oder 7, **dadurch gekennzeichnet, dass** der Schraubeinsatz (20) durch ein Klemmelement (23) in dem Frontstück (9.1) festlegbar ist.

## Claims

1. A handpiece for in particular medical laser applications, with an inner channel (6, 8, 8.1) into which a laser fibre (7) is inserted which projects with a free end section of a length (L) via a channel outlet (10), the length (L) of the laser fibre (7) which emerges from the channel outlet (10) being changeable, it consisting at least of two sections (1, 1.1, 9, 9.1, 20), the position of which relative to each other is changeable, **characterised in that** latch teeth (26) are provided on or in one section, into which teeth engages an actuatable latch lever (27) which is designed as a rocker lever which is fixed on the other section.

2. A handpiece according to Claim 1, **characterised in that** the change in position takes place by axial displacement of at least two sections (1, 1.1, 9) relative to each other.

3. A handpiece according to one of the preceding claims, **characterised in that** a cylindrical connection piece (1) has on the periphery of an plug-in sleeve (12) a plurality of crenellation-like latch rings (13), with which elastic elements (14) are associated being arranged in a push-on sleeve (16) of a front piece (9), which elastic elements engage between the latch rings (13).

4. A handpiece according to Claim 3, **characterised in that** the elastic elements are rubber rings (14) which lie in annular grooves (15) in the push-on sleeve (16).

5. A handpiece according to one of the preceding claims, **characterised in that** the change in position takes place by turning of at least two sections (9.1, 20) relative to each other.

6. A handpiece according to one of the preceding claims, **characterised in that** a screw insert (20) is inserted into a front piece (9.1), which insert also has the channel outlet (10).

7. A handpiece according to Claim 6, **characterised in that** the screw insert (20) by means of an external thread (21) meshes with an internal thread (22) in the front piece (9.1).

8. A handpiece according to Claim 6 or 7, **characterised in that** the screw insert (20) can be fixed in the front piece (9.1) by a clamping element (23).

## Revendications

1. Pièce à main en particulier pour applications médicales utilisant un laser, avec un canal intérieur (6, 8, 8.1) dans lequel est placé une fibre laser (7) qui ressort, par un segment d'extrémité libre d'une longueur (L), d'une embouchure de canal (10), la longueur (L) de la fibre laser (7) ressortant de l'embouchure de canal (10) étant variable,
la pièce étant composée d'au moins deux parties (1, 1.1, 9, 9.1, 20) dont la position l'une par rapport à l'autre est variable,
**caractérisée par le fait que** sur ou dans une partie est prévue une denture d'encliquetage (26) dans laquelle engrène un levier d'encliquetage (27) actionnable qui est réalisé sous forme de levier pivotable fixé à l'autre partie.

2. Pièce à main selon la revendication 1, **caractérisée par le fait que** le changement de position a lieu par un déplacement axial d'au moins deux parties (1, 1.1, 9) l'une par rapport à l'autre.

3. Pièce à main selon l'une des revendications précédentes, **caractérisée par le fait qu'**une pièce de raccordement cylindrique (1) sur le pourtour d'un manchon enfichable (12) présente une pluralité de bagues d'encliquetage en forme de créneaux (13) auxquelles sont associés, dans un manchon emboîtable (16) d'une pièce frontale (9), des éléments élastiques (14) qui s'engagent entre les bagues d'encliquetage (13).

4. Pièce à main selon la revendication 3, **caractérisée par le fait que** les éléments élastiques sont des bagues en caoutchouc (14) qui se trouvent dans des rainures annulaires (15) dans le manchon emboîtable (16).

5. Pièce à main selon l'une des revendications précédentes, **caractérisée par le fait que** le changement de position a lieu par un décalage d'au moins deux parties l'une par rapport à l'autre (9.1, 20).

6. Pièce à main selon l'une des revendications précédentes, **caractérisée par le fait que** dans une pièce frontale (9.1) est placé un insert de vis (20) qui présente également l'embouchure de canal (10).

7. Pièce à main selon la revendication 6, **caractérisée par le fait que** l'insert de vis (20) engrène par un filet extérieur (21) un filet intérieur (22) dans la pièce frontale (9.1).

8. Pièce à main selon la revendication 6 ou 7, **caractérisée par le fait que** l'insert de vis (20) peut être fixé, par un élément de serrage (23), dans la pièce frontale (9.1).
